# EUROPÄISCHE PATENTSCHRIFT

(11) **EP 0 981 377 B1**
(45) Veröffentlichungstag und Bekanntmachung des Hinweises auf die Patenterteilung: **10.09.2003**
(21) Anmeldenummer: 98914877.0
(22) Anmeldetag: 11.03.1998
(51) Int. Cl.: A61K 47/48

(54) **KOMPLEXE DES LHRH-ANTAGONISTEN CETRORELIX MIT POLYAMINOSÄUREN SOWIE VERFAHREN ZU DEREN HERSTELLUNG**
cOMPLEXES OF THE LHRH-ANTAGONIST CETRORELIX WITH POLY-AMINO ACIDS AND METHOD FOR THEIR PRODUCTION
COMPLEXES DE L'ANTAGONISTE DE LH-RH CETRORELIX AVEC DES ACIDES POLYAMINES ET PROCEDE POUR LEUR PREPARATION

(30) Priorität: 26.03.1997 DE 19712718
(43) Veröffentlichungstag der Anmeldung: 01.03.2000
(73) Patentinhaber: Zentaris AG, 60314 Frankfurt/Main (DE)
(72) Erfinder: ENGEL, Jürgen, D-63755 Alzenau (DE); DEGER, Wolfgang, D-60389 Frankfurt (DE); REISSMANN, Thomas, D-60347 Frankfurt (DE); LOSSE, Günter, D-01069 Dresden (DE); NAUMANN, Wolfgang, D-09600 Zug (DE); MURGAS, Sandra, D-01187 Dresden (DE)
(86) Internationale Anmeldenummer: EP9801398
(87) Internationale Veröffentlichungsnummer: WO98042381

(56) Entgegenhaltungen:
- EP-A- 0 371 195
- EP-A- 0 611 572
- WO-A-94/11015
- WO-A-94/27641
- DD-A- 269 785
- US-A- 4 341 767
- US-A- 5 502 035
- REISSMANN TH ET AL: "DEVELOPMENT AND APPLICATIONS OF LUTEINIZING HORMONE-RELEASING HORMONE ANTAGONISTS IN THE TREATMENT OF INFERTILITY: AN OVERVIEW" HUMAN REPRODUCTION, Bd. 10, Nr. 8, August 1995, Seiten 1974-1981, XP002037921

## Beschreibung

Die Erfindung betrifft aktivitätsstabilisierte und retardierende Komlexe eines LHRH-Antagonisten ,nämlich Cetrorelix, mit den Polyaminosäuren Poly-Glutaminsäure und Poly-Asparaginsäure sowie Verfahren zur Herstellung derselben und diese enthaltende Arzneimittel.
Die dargestellten Peptidhormon-Polyaminosäure-Komplexe können in der Medizin beispielsweise zur Therapie hormonsensitiver Tumore, wie z.B. Mamma- und Prostatakarzinome, der benignen Prostatahypertrophie und in der Gynäkologie zur Behandlung der Endometriose, Hysteroskopie und zur Behandlung von Fertilitätsstörungen Verwendung finden.

In den Patentschriften DD 257197, DD 269785 und DD 299265 werden für Insulin und für andere biologisch aktive Proteohormone Verfahren zur Herstellung von immobilisierten, in ihren biologischen Aktivitäten stabilisierten und in ihren pharmakologischen Eigenschaften modifizierten Peptidpräparaten beschrieben, deren wesentlichstes Merkmal die Komplexbildung des jeweiligen Peptides mit Polyaminosäuren ist.
In den angeführten Patenten werden Präparationsverfahren beschrieben, bei denen die Komplexe unter Einwirkung von Ameisensäure sowie organischen Lösungsmitteln wie Chloroform und drastischen Herstellungsbedingungen gebildet werden. Dabei besteht für das Peptidhormon die Gefahr der partiellen Inaktivierung und verminderten Stabilität.
In der Literatur wurden 1981 erstmalig in den EP 0042753 und EP 0049628 schwerlösliche Salze oder Komplexe für LHRH-Analoga beschrieben. Die Darstellung dieser Komplexe erfolgte in Hinblick auf die Entwicklung pharmazeutischer Produkte für unterschiedliche medizinische Applikationen.

1989 beschreib ORSOLINI im DE Patent 38 22 459 die Herstellung wasserunlöslicher Polypeptide durch Komplexbildung von LHRH-Analoga mit Embonsäure, Tannin und Stearinsäure. Die erhaltenen schwerlöslichen Komplexe werden dabei zusätzlich in eine polymere Matrix aus (Milchsäure-Glycolsäure)-Copolymer eingebettet.
Weitere Verfahren zur Herstellung von Cetrorelix--Komplexes eingebettet in ein (Milchsäure-Glycolsäure)-Copolymer, wurden 1993 von ORSOLINI und HEIMGARTNER in den DE Patenten 42 23 282 und 42 23 284 beschrieben. In diesem Patent schwerlösliche Cetrorelixkomplexe mit Embonsäure,Tannin, Stearinsäure und Palmitinsäure aufgeführt.

Aus US 4,341,767 ist bekannt, LHRH-Antagonisten von Nona- und Dekapeptiden als Arzneiform mit verzögerter Freisetzung des Wirkstoffes bereitzustellen, indem die Peptide als Säureadditionssalze mit einer polybasischen Säure verwendet werden.

Auch US 5,502,035 beschreibt Deka-und Undekapeptide als LHRH-Antagonisten, die als Arzneiform mit verzögerter Freisetzung des Wirkstoffes anwendbar sind, wobei die Peptide als Säureadditionssalze mit einer polybasischen Säure eingesetzt werden.

Ziel der Erfindung ist es, Depotpräparate mit verbesserten und steuerbaren Retardeigenschaften und erhöhter Stabilität gegenüber vorzeitigem proteolytischem Abbau eines LHRH-Antagonisten zur Therapie auf den hierfür bekannten Gebieten wie hormonsensitiver Tumore, wie z.B. Mamma- und Prostatakarzinome, der benignen Prostatahypertrophie, Endometriose, Hysteroskopie und zur Behandlung von Fertilitätsstörungen bereitzustellen und einfach beherrschbares und umweltfreundliches Verfahren zur Herstellung dieser Präparate anzugeben.
Die Aufgabe der Erfindung besteht darin, Depotpräparate mit verbesserten und steuerbaren Retardeigenschaften des LHRH-Antagonisten Cetrorelix mit biologisch abbaubaren Polymeren bereitzustellen, sowie ein Verfahren zu deren Herstellung anzugeben

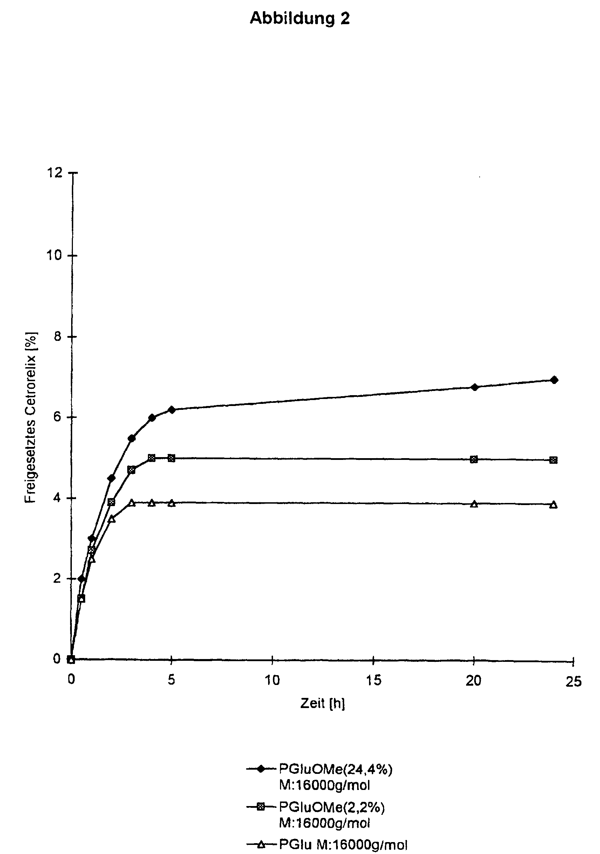

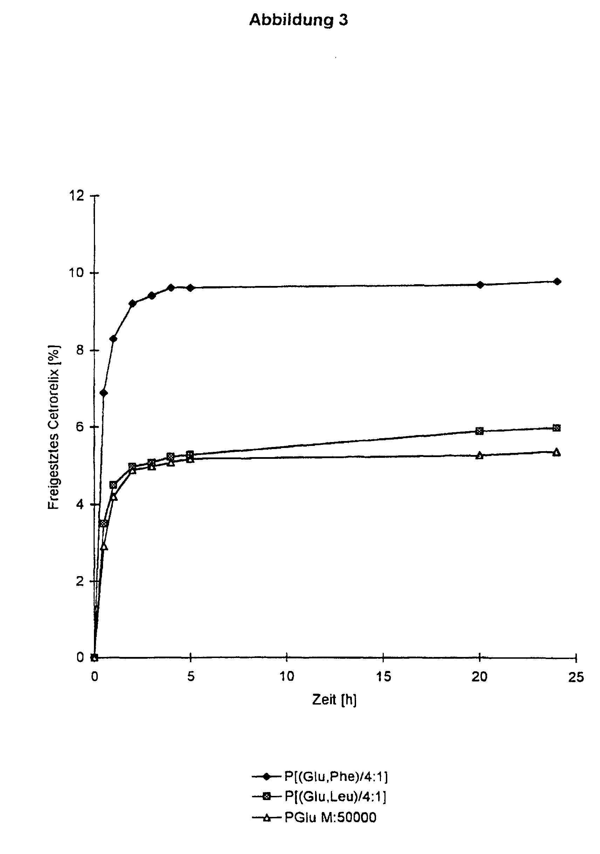

Erfindungsgemäß wird die Aufgabe dadurch gelöst, daß immobilisierte und aktivitätsstabilisierte, parenteral applizierbare Peptidhormonpräparate aus Komplexen des LHRH-Antagonisten Cetrorelix mit Polyaminosäuren aus der Gruppe bestehend aus Poly-Glutaminsäure und Poly-Asparaginsäure hergestellt werden, indem der Polyaminosäure-Peptidhormon-Komplex unter Vermeidung organischer Lösungsmittel aus wäßrigen Lösungen gefällt wird. Vorteilhaft können ferner die Polyaminosäure-Peptidhormon-Komplexe mit einem steuerbaren Hormongehalt durch Lyophilisation der wäßrigen Lösungen hergestellt werden. Durch die Art und die Molmasse der Polyaminosäuren, durch Einbau hydrophober Aminosäuren in das Polymergerüst oder durch partielle Veresterung läßt sich die Freisetzungsrate des Wirkstoffes steuern.(Abb. 2 und 3)

Die Verwendung der erfindungsgemäßen Komplexe erfolgt in der Medizin zur Therapie hormonsensitiver Tumore, insbesondere zur Behandlung von Mammaund Prostatakarzinomen, der benignen Prostatahypertrophie sowie in der Gynäkologie zur Ovulationsinduktion, Invitro-Fertilisation und Endometriose und in Verbindung mit der Hysteroskopie.

Der Begriff "Komplex" umfaßt im Rahmen dieser Erfindung die Zusammenlagerung zweier oder mehrerer Komponenten zu einem schwerlöslichen System, das keiner nachgewiesenen Stöchiometrie unterliegt. Dabei kommt es zu einer Überlagerung von Wechselwirkungen,
wobei hauptsächlich Nebenvalenzbindungen eine Rolle spielen.
In der Literatur werden schwerlösliche Peptidkomplexe mitunter auch als "Salz" bezeichnet.
Diese Bezeichnung ist ebenfalls in vielen Fällen nicht exakt, da es sich nicht, wie bereits erwähnt, um Substanzen mit definierter Zusammensetzung handelt.
Bei Peptiden und Proteinen treten zwar ionische Wechselwirkungen auf, doch sie sind nicht allein für eine Struktur- oder Aggregatzustandsänderung verantwortlich.
Für Peptide und Proteine ist der Begriff "Komplex" und "Salz" aufgrund der Vielzahl der funktionellen Gruppen weiter zu fassen, da sich mehrere Wechselwirkungen, die zu Aufbau und Struktur der Peptide und Proteine führen, überlagern.

Es wurden Polyaminsäuren verwendet, die sich als biophile Trägermaterialien für Peptide eignen. Erfindungswesentlich ist dabei, daß die Wirkstoffe nicht Kovalent an das Polymer gebunden werden, sondern lediglich durch Nebenvalenzbindungen und hydrophobe Wechselwirkungen an das Polymer fixiert sind.
Unvermutet zeigte sich, daß speziell der LHRH-Antagonist Cetrorelix eine sehr hohe Bindungsaffinität zu Poly-Glutaminsäure und Poly-Asparaginsäure, aufweist. Eine solch hohe Affinität von Cetrorelix war auf Grund des bisherigen Literaturstandes nicht vorhersehbar und auf Grund der Struktur des Peptides überraschend.
Die spontan ausfallenden Komplexe besitzen einen definierten, reproduzierbaren Hormongehalt.
Soll der Hormongehalt in den Komplexen dagegen variieren und genau festgelegt sein, so hat sich die Lyophilisation als geeignete Methode herausgestellt.
Diese Herstellungsbedingungen sind wesentlich milder als in früheren Patenten beschriebene und verhindern somit eine mögliche Inaktivierung des Hormons.
Die beim Vermischen der Lösungen auftretenden Wechselwirkungen zwischen den Molekülen führen zu stabilen Komplexen, die ein steuerbares Wirkstoffabgabeprofil und eine erhöhte Proteolysestabilität aufweisen.
Polyaminosäuren beeinflussen somit nicht nur das Retardverhalten, sondern bieten gleichzeitig Schutz vor unerwünschtem, vorzeitigen proteolytischen Abbau. Dieser Aspekt ist vor allem im Hinblick auf den Langzeiteinsatz solcher Praparate von Vorteil.

Das Retardverhalten der Komplexe kann im Wesentlichen durch die Art und die Molmasse der Polyaminosauren, den Einbau von Aminosauren mit hydrophoben Seitenketten in das Polymergerust und durch partielle Veresterung vorhandener Carboxylgruppen beeinflußt werden.

Die Erfindung wird an Hand der nachfolgenden Ausfuhrunsgbeispiele näher erläutet ohne sie jedoch einzuschränken.

Herstellung von Polyaminosaure-Peptid-Komplexen durch Fällung

### Beispiel 1

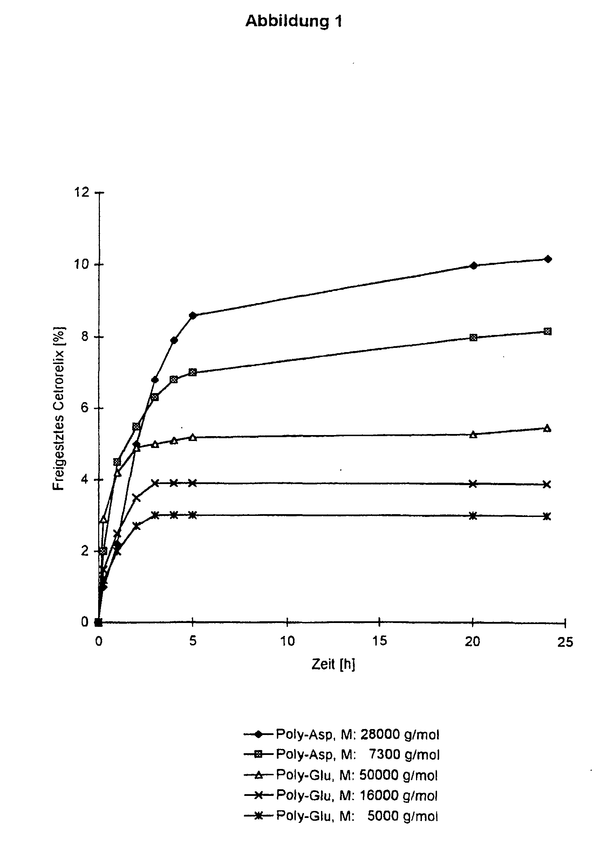

50 mg Polyaminosäure werden in 5 ml H₂O, bei Poly-Glu unter Zusatz von IN NH₄OH, leichter Erwärmung auf 40 °C und Ultraschallbehandlung, gelost 50 mg Cetrorelix (als Azetat) werden in 4 ml H₂O gelost. Die Polyaminosäure-Lösung wird gerühert und die Cetrorelixlösung in einem Schritt zugegeben und anschließend 4 Std. bei 4°C aufbewahrt. Danach wird der Niederschlag 5 min bei 4000 rpm abzentrifugiert, der Überstand abgenommen und das Präzipitat 24 Stunden im Vakuum über P₂O₅ getrocknet. Da keine stöchiometrischen Komplexe vorliegen, wurde die Ausbeute auf die Summe der Ausgangsstoffe bezogen. Abbildung 1 zeigt verschiedene Freisetzungskurven im statistischen Liberationssystem in Abhängigkeit von der Molmasse. (Freisetzungsmedium 0,01 m Amoniumazetat, pH 7,0)

### Ausbeute: 50-65 % d.Th.

### Cetrorelixgehalt im Komplex: siehe Tabelle 1

**Tab.1:**

| Zusammensetzung der gefällten Cetrorelix-Polyaminosaure-Komplexe | | | | |
|---|---|---|---|---|
| Polyaminosäure | Mittlere Molmasse [g/mol] | Hormongchalt im Komplex [%] rel. Fehlcr: 5% | Molverhätnis Hormon : PAS | Molverhältnis Hormon : freie Carboxylgruppen |
| Poly-Glutaminsäure | 5000 | 86 | 1 : 0,05 | 1 : 1,9 |
| | 16000 | 85 | 1 : 0,016 | 1 : 2,1 |
| | 50000 | 60 | 1 : 0,02 | 1 : 8,2 |
| Poly-Glutaminsäuremethylester Methylierungsgrad: | | | | |
| 2,2% 24,4% | 16000 | 81 | 1 : 0,02 | 1 : 2,8 |
| | 16000 | 58 | 1 : 0,06 | 1 : 6,7 |
| Poly-Asparaginsaure | 7300 | 86 | 1 : 0,03 | 1 : 2,2 |
| | 14000 | 78 | 1 : 0,03 | 1 : 3,8 |
| | 28000 | 69 | 1 : 0,023 | 1 : 6,1 |
| Poly[(Glu,Phe)/4:1] | 45000 | 65 | 1 : 0,017 | 1 : 4,5 |
| Poly[(Glu,Leu)/4:1] | 70000 | 79 | 1 : 0,005 | 1 : 2,4 |

Herstellung von Polyaminosäure-Peptid-Komplexen mit definiertem Peptidgehalt durch Lyophilisation

### Beispiel 2

### Cetrorelix- Komplex mit 50%igem Peptid - Gehalt

50 mg Polyaminosaure werden in 5 ml H₂O, bei Poly-Glu unter Zusatz von 1_{N} NH₄OH, leichter Erwärmung auf 40°C und Ultraschallbehandlung, gelöst, 50 mg Cetrorelix (als Azetat) werden in 4 ml H₂O gelost Die Polyaminosäure -Lösung wird gerühert und die Cetrorelixlösung mit einem Mal zugegeben und weitere 2 min gerühert. Der entstandene Komplex wird bei -20°C eingefroren und im Anschluß lyophilisiert. Da keine stöchiometrischen Komplexe vorliegen, wurde die Ausbeute auf die Summe der Ausgangsstoffe bezogen

### Ausbeute : 90-95 % d. Th

### Cetrorelixgehalt im Komplex : 45-50 %

### Beispiel 3

Durch entsprechende Abwandlung der Menge an Polyaminosaure und Cetrorelix wurde in analoger Weise ein 70% iger Cetrorelix-Komplex hergestellt

### Beispiel 4

Eine Erhöhung der Hydrophobizitat,verbunden mit einer Zunahme des Retardverhaltens kann u.a. durch die partielle Veresterung von Carboxylgruppen erreicht werden. In Abb.2 sind die Freisetzungskurven von Cetrorelixkomplexen mit Poly-Glutaminsäuremethylestern dargestellt. Abb. 3 zeigt die Freisetzungskurven der Cetrorelixkomplexe der Polyglutaminsaure
mit Leucin und Phenylalanin.

### Beispiel 5

Zur Überprüfung der in vitro-Freisetzungsversuche wurden Cetrorelix-Polyaminosäure-Komplexe im Tierversuch getestet.
Es handelt sich dabei um die Cetrorelixkomplexe mit folgenden Polyaminosäuren:
― Poly-Glutaminsäure, M: 5000 g/mol
― Poly-Glutaminsaure, M: 16000 g/mol
― Poly-Asparaginsäure, M. 7300 g/mol

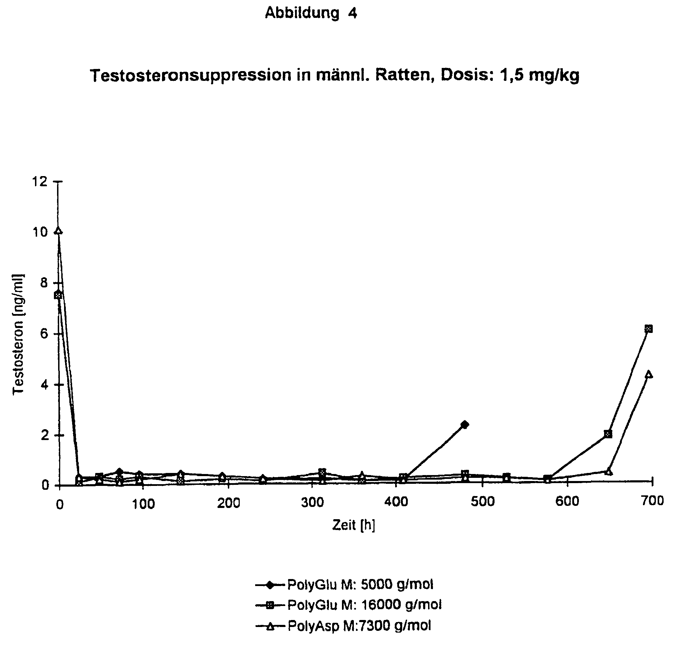

In Abb.4 ist die Testosteronsuppression bei männlichen Ratten nach einmaliger s.c. Injektion von 1,5 mg/kg dargestellt. Je Versuchsgruppe wurden 5 Tiere getestet.

Mit diesen Ergebnissen konnte gezeigt werden, daß die untersuchten Komplexe einen Langzeiteffekt in der Testosteronsuppression über 600 Stunden bewirken.(Abb. 4)
Es wurde die prinzipielle Wirksamkeit und Eignung der Cetrorelix-Polyaminosäure-Komplexe als Depotpräparat nachgewiesen.

## Patentansprüche

1. Komplexe des LHRH-Antagonisten Cetrorelix mit Polyaminusäuren aus der Gruppe bestehend aus Poly-Glutammsäure und Poly-Asparaginsäure einer mittleren Molmasse der Polyaminosäuren von 2000-20.000 g/mol.

2. Verfahren zur Herstellung von immobilisierten und aktivitätsstabilisierten, parenteral applizierbaren Peptidhormonpräparaten aus Komplexen des LHRH-Antagonisten Cetrorelix mit Poly-Glutaminsäure und Poly-Asparaginsäure der mittleren Molmassen 2000-20.000 g/mol nach Anspruch 1, **gekennzeichnet dadurch, dass** der Polyaminosäure-Peptidhormon-Komplex aus wässrigen Lösungen gefällt wird.

3. Verfahren nach Anspruch 2, **dadurch gekennzeichnet, dass** die Polyaminosäure -Peptidhormon-Komplexe mit einem gewünschten steuerbaren Hormongehalt durch Lyophilisation der wässrigen Lösungen hergestellt werden.

4. Verfahren nach Anspruch 2, **dadurch gekennzeichnet, dass** man durch Einbau hydrophober Aminosäuren in das Polymergerüst oder durch partielle Veresterung die Freisetzungsrate des Wirkstoffes steuert.

5. Arzneimittel **gekennzeichnet durch** einen Gehalt einer oder mehrerer Verbindungen nach den Ansprüchen 1-4 sowie gegebenfalls Hilfs- und Gerüststoffen sowie Stabilisatoren .

6. Arzneimittel zur Verwendung bei der Therapie hormonsensitiver Tumore, insbesondere zur Behandlung von Mamma-, Ovarial- und Prostatakarzinomen, der benignen Prostatahypertrophie, von Fertilitätsstörungen, der Endometriose und in Verbindung mit der Hysteroskopie sowie zur Invitro-Fertilisation **gekennzeichnet durch** einen Gehalt an Verbindungen nach Anspruch 1-4.

## Claims

1. Complexes of the LHRH antagonist cetrorelix with polyamino acids selected from the group consisting of polyglutamic acid and polyaspartic acid of an average molecular mass of the polyamino acids of 2000-20000 g/mol.

2. Process for the preparation of immobilized and activity-stabilized, parenterally administerable peptide hormone preparations from complexes of the LHRH antagonist cetrorelix with polyglutamic acid and polyaspartic acid of average molecular masses 2000-20000 g/mol according to Claim 1, **characterized in that** the polyamino acid-peptide hormone complex is precipitated from aqueous solutions.

3. Process according to Claim 2, **characterized in that** the polyamino acid-peptide hormone complexes having a desired controllable hormone content are prepared by lyophilization of the aqueous solutions.

4. Process according to Claim 2, **characterized in that** the release rate of the active compound is controlled by incorporation of hydrophobic amino acids into the polymer structure or by partial esterification.

5. Medicaments **characterized in that** they contain one or more compounds according to Claims 1-4, and optionally auxiliaries and bulking agents as well as stabilizers.

6. Medicaments for use in the therapy of hormone-sensitive tumours, in particular for the treatment of breast, ovarian and prostate carcinomas, benign prostate hypertrophy, of fertility disorders, endometriosis and in connection with hysteroscopy, and for in vitro fertilization **characterized in that** they contain compounds according to Claim 1-4.

## Revendications

1. Complexes de l'antagoniste de LHRH Cetrorelix avec des polyaminoacides choisis dans le groupe constitué par les acides polyglutamiques et par les acides polyaspartiques, ayant une masse molaire moyenne des polyaminoacides de 2 000 à 20 000 g/mol.

2. Procédé de préparation d'hormones peptidiques administrables par voie parentérale, ayant une activité stabilisée à base de complexe d'antagoniste de LHRH Cetrorelix, avec des acides polyglutamiques et polyaspartiques ayant une masse molaire moyenne de 2 000 à 20 000 g/mol selon la revendication 1,
**caractérisé en ce que**
le complexe polyaminoacide - hormone peptidique est précipité de solutions aqueuses.

3. Procédé selon la revendication 2,
**caractérisé en ce que**
les complexes polyaminoacides - hormone peptidiques sont produits avec une teneur en hormone contrôlable désirée, par lyophilisation des solutions aqueuses.

4. Procédé selon la revendication 2,
**caractérisé en ce qu'**
on contrôle par incorporation d'aminoacides hydrophobes dans le squelette de polymère ou par estérification partielle, la vitesse de libération du principe actif.

5. Médicaments **caractérisés par** une teneur en un ou plusieurs composés selon les revendications 1-4 ainsi qu'éventuellement en substances auxiliaires et des substances de structuration ainsi qu'agents stabilisants.

6. Médicaments destinés à l'utilisation dans la thérapeutique de tumeurs hormono-sensibles en particulier pour le traitement des carcinomes du sein, des ovaires et de la prostate, de l'hypertrophie bénigne de la prostate, des troubles de la fertilité, de l'endométriose et en liaison avec l'hystéroscopie ainsi que pour la fertilisation in vitro,
**caractérisés par** une teneur en composés selon la revendication 1 à 4.
